# EUROPEAN PATENT APPLICATION

(11) **EP 4 559 493 A1**
(43) Date of publication of application: **28.05.2025**
(21) Application number: 24214524.1
(22) Date of filing: 21.11.2024
(51) Int. Cl.: A61M 5/142, A61M 5/162

(54) **WEARABLE PUMP INFUSION SYSTEM**

(30) Priority: 22.11.2023 US 202363601881 P; 22.11.2023 US 202363601882 P; 20.12.2023 US 202363612658 P
(71) Applicant: TxSphere LLC, Natick Massachusetts 01760 (US)
(72) Inventor: Mao, Zhenhua, Andover, 01810 (US)
(74) Representative: J A Kemp LLP

(57) **Abstract**

A wearable pump infusion assembly includes a housing to selectively support a vial containing a medicament for delivery to a patient and a patient support member to support the housing on the patient. A bottle connector, mounted within the housing, includes a hollow needle to puncture a seal on the vial and to receive the medicament from the vial and a connector in fluid communication with the hollow needle. The wearable pump infusion assembly also includes a flexible IV tube and a peristaltic pump. The flexible IV tube has a first end connected to the connector, a second end connected to another connector, and a central portion extending between the first and second ends. The peristaltic pump includes multiple plunger elements selectively engaging the central portion of the flexible IV tube to compress and release the central portion of the flexible IV tube to draw the medicament from the vial.

## Description

### CROSS REFERENCE TO RELATED APPLICATION

This application claims priority to U.S. Provisional Application Ser. No. 63/601,881, entitled Hand-Held Ambulatory Pump Infusion System, filed November 22, 2023; U.S. Provisional Application Ser. No. 63/601,882, entitled Clip-On Pump Infusion System, filed November 22, 2023; and U.S. Provisional Application Ser. No. 63/612,658, entitled Wearable Pump Infusion System, filed December 20, 2023, the entire contents of each application is incorporated herein by reference.

### BACKGROUND OF THE INVENTION

### Field of the Invention

The present invention relates to systems for intravenous (IV) administration of drugs and, in particular, to a pump system allowing improved flow-control of IV bag or bottle medicines used in extra-institutional settings.

### Description of Related Art

Medical pumps, such as infusion pumps, are known for computer-controlled delivery of medication, vitamins, minerals, or other nutrients or supplements in a solution (henceforth medicaments) to patients over a period of time. One type of infusion pump is an intravenous (IV) pump. Precise delivery of IV medicaments to a patient may be provided by an IV pump, for example, supported on a pole stand holding an elevated IV bag or bottle. An IV line from the IV bag or bottle is threaded through the pump to communicate with a needle terminating the IV line and introduced into the patient. Operation of the IV pump can then provide a controlled metering of the IV fluid.

Portable IV pumps that can provide similar metering are also known, for example, in the delivery of medicaments to a patient in an ambulatory setting. Such pumps include a battery providing power to the pump and mobility to the patient. In some cases, the pump may be sufficiently light to be worn by the patient, for example, on a belt or the like also holding the IV bag or bottle.

When IV fluids need to be delivered outside of an institutional setting, conventional gravity feed of the IV fluid may be relied upon, with the height of the IV bag or bottle adjusted to obtain a desired flow rate, for example, visually monitored by counting drops of liquid in a clear chamber attached to the IV line.

US patent application 17/520,182, entitled "Clip-On Flow Control for IV Lines," filed Nov. 5, 2021, hereby incorporated by reference, describes a "clip-on" flow controller for IV lines which automatically controls flow rate over a range of pressures through a self-contained valve and flow sensor. By eliminating a pump, a lightweight system with a long operating life can be produced, as well as a system that can be self-supported on the IV line yet still provide institutional-level flow accuracy without accurate elevation of the IV bag or careful monitoring.

Still other infusion pumps besides IV pumps may be provided to deliver medicaments to patients. Recently, battery-powered compact infusion pumps have become available that permit the patient to remain active (ambulatory), for example, in a home environment away from a clinic or hospital during the treatment.

Typically, the medicament is provided in a drug container, e.g., IV bag or bottle/vial, that may be connected to an IV line which in turn attaches to a needle or port communicating with the patient. A nurse or other health care professional ministering to the patient receives the medicament, reviews the medicament description for correctness, and enters the desired dose and rate into the pump. The IV line is then installed in the portable pump and the assembly placed in a pack or other carrying apparatus that may be retained on the patient. The medicament may be delivered as the patient proceeds through normal life activities until the full dose is complete. The patient may then return the assembly to the nurse or health care professional who may provide a new bag of medicament and IV line and may reprogram the pump for new treatment.

Pumps suitable for ambulatory use can have high total operating costs driven in part by the cost of medical care required to have nurses or health care professionals review the medicament, program the pump with the prescribed dose and rate, and load the IV line cartridge onto the portable pump for every drug delivery. This process is repeated for every new container of medicament that needs to be loaded increasing the cost of travel, either by the patient or the health care professional, between the hospital or clinic and the home of the patient.

Also, the need for high precision can require heavy and extensive plastic IV line cartridges or pumps that work against the costs of delivering medicine with patient loaded IV pumps. Heavy and extensive plastic materials can also make it difficult to install and remove the replaceable IV line cartridges which need high forces to compress the IV line and release anti-free flow locks to install the IV line properly.

US patent 10,869,963, entitled "Low-cost ambulatory medical pump," hereby incorporated by reference, describes a low-cost medical pump for ambulatory use provides reduced life components combined with a pump lockout enforcing a safe operating limit and preventing reuse after that limit is exceeded. An improved IV line clamp portion minimizes unsupported clamp structure length and provides a dual lock system preventing inadvertent clamp release.

US patent 11,712,512, entitled "Ambulatory medical pump cartridge locking system," hereby incorporated by reference, describes a low-cost medical pump for ambulatory use which provides an improved IV line cartridge attachment allowing for single handed, low force release while preventing inadvertent IV line cartridge release. A rotatable side lever minimizes the force required to move the locking elements against a spring biased force urging the locking elements to a locking position.

### SUMMARY OF THE INVENTION

The present inventors have determined that the ability for the patient to install their own drug containers and IV line to the portable pump can greatly reduce healthcare professional time and patient travel costs. For example, home installation may be desired in situations when the patient cannot easily travel to the hospital or clinic and/or when access to health care professionals for at-home visits is limited, such as patients living in remote locations. In these situations, it is useful for the new containers of medicament and IV line to be shipped or delivered to the patient's home where the patients may self-install the new containers of medicament and IV line without the assistance of a health care professional.

The present invention provides an ambulatory pump intended to increase availability and acceptability of ambulatory infusion for patient loaded IV pumps. The pump has separable housing components, (1) a reusable pump housing with the significant mechanical component of the pump and (2) a disposable drug holder that supports the preloaded drug containers, and an IV tube cartridge with an IV tube that can be constructed with less material reducing operating costs. Good dimensional stability can be achieved by mounting the connectors of the IV tube cartridge and pump housing, respectively, at opposed long edges of the housing facilitating precise alignment of the IV tube and pumping elements.

The ambulatory pump is connectable to one or more disposable drug containers, e.g., IV bottles or vials, held within a disposable drug holder. The disposable drug containers are held by the lightweight rigid housing of the drug holder facilitating attachment of the disposable drug holder to the ambulatory pump. The disposable drug holder may hold one or more disposable drug containers of different sizes, i.e., large and small, to be connectable to an IV tube communicating with the pump elements of the ambulatory pump to pump the fluid from the drug container through the IV tube. The disposable drug holder supports a pump connector that is an IV tube cartridge with an IV tube guide channel holding an IV tube that easily snaps onto the bottom of the ambulatory pump to promote peristaltic pumping of the liquid medicine through the IV tube. The opposed ends of the IV tube may include a drug container connector on a first end providing one way flow of the liquid medicine from the container and an IV tube connector on a second end providing connection of the liquid medicine to an IV tube terminating at the patient through a needle or port or the like.

A lightweight pump system with a long operating life can be produced, as well as a system that can be self-supported on the IV line yet still provide institutional-level flow rate accuracy without accurate elevation of the IV container or careful monitoring. A simplified peristaltic pump allows for precise flow control that can be comfortable worn by the patient with built in flow rate sensing. The disposable IV tube cartridge is self-supported on the IV line, allowing the drug container to be worn, for example, around the patient's neck in an inverted position to take advantage of natural gravity feed. The peristaltic pump can be removed from the drug holder for reuse with different drug containers/drug holder. In certain embodiments, the drug holder is disposable and may be disposed with the drug containers after use. Thus, the peristaltic pump can be reused while the drug container components are one time use, minimizing the overall system cost.

According to one embodiment of the invention, a wearable pump infusion assembly includes a housing configured to selectively support a vial containing a medicament for delivery to a patient, a patient support member to removably mount the housing on the patient, and a bottle connector mounted within the housing. The bottle connector includes a hollow needle to puncture a seal on the vial as the vial is inserted into the housing and to receive the medicament from the vial. The bottle connector also includes a connector in fluid communication with the hollow needle to receive the medicament from the vial. The pump infusion assembly also includes a flexible IV tube and a peristaltic pump. The flexible IV tube has a first end connected to the connector on the bottle connector, a second end connected to another connector, and a central portion extending between the first end and the second end. The peristaltic pump includes multiple plunger elements selectively engaging the central portion of the flexible IV tube to compress and release the central portion of the flexible IV tube to draw the medicament from the vial. The patient support member may be a neck strap, a belt, or a clip.

According to another aspect of the invention, the wearable pump infusion assembly includes an opening defining, at least in part, a fluid communication path between an ambient environment outside the vial and an interior volume of the vial. A hydrophobic filter is provided in the fluid communication path to permit air from the ambient environment into the vial and to prevent the medicament from exiting the opening.

According to still another aspect of the invention, the wearable pump infusion assembly includes a locking assembly to engage a neck of the vial, to draw the vial onto the hollow needle of the bottle connector, and to secure the vial within the bottle connector. The locking assembly includes a first hinged latch on a first side of the housing and a second hinged latch on a second side of the housing, the second side opposite the first side.

According to yet other aspects of the invention, the housing of the wearable pump infusion assembly includes a first portion and a second portion. The first portion has a first interlocking element, at least one vial retaining member to receive the vial, the bottle connector mounted within the housing, and the flexible IV tubing mounted therein. The second portion includes a second interlocking element, complementary to the first interlocking element, where the first and second interlocking elements hold the first and second portions of the housing together. The second portion of the housing also includes an electronics housing, where a controller for the wearable pump infusion assembly is contained within the electronics housing. The second portion of the housing may be reusable with multiple first portions of the housing.

According to still other aspects of the invention, the electronics housing includes at least one selection device to adjust a desired flow rate of the medicament from the vial and at least one flow rate sensor to detect an actual flow rate of the medicament from the vial. The controller is operative to receive a feedback signal from the at least one flow rate sensor and to adjust a rate at which the plunger elements engage the central portion of the flexible IV tube as a function of the feedback signal to achieve the desired flow rate. The selection device may be at least one selection button activated by the patient to adjust the desired flow rate. Optionally, the selection device may be at least one selection switch within the electronics housing to adjust the desired flow rate.

According to another embodiment of the invention, a method for delivering a medicament to a patient includes inserting a vial containing the medicament for delivery to the patient into a housing. A bottle connector is mounted within the housing and the bottle connector includes a hollow needle to puncture a seal on the vial as the vial is inserted into the housing and to receive the medicament from the vial and a connector in fluid communication with the hollow needle to receive the medicament from the vial. A patient support member is placed on the patient to support the housing on the patient. A button on a user interface is pressed to initiate treatment of the patient, and the medicament is delivered to the patient via a peristaltic pump and a flexible IV tube. The flexible IV tube has a first end connected to the connector, a second end connected to another connector, and a central portion extending between the first end and the second end. The peristaltic pump includes multiple plunger elements selectively engaging the central portion of the flexible IV tube to compress and release the central portion of the flexible IV tube to draw the medicament from the vial.

These particular objects and advantages may apply to only some embodiments falling within the claims and thus do not define the scope of the invention.

### BRIEF DESCRIPTION OF THE DRAWINGS

Various exemplary embodiments of the subject matter disclosed herein are illustrated in the accompanying drawings in which like reference numerals represent like parts throughout, and in which:
FIG. 1 is an exemplary environmental view of the wearable pump assembly communicating with an inverted drug container suspended from a patient's neck and an electronic component attached to an IV tube cassette to form the clip-on pump housing;
FIG. 2 is an exploded, enlarged view of the clip-on pump of Fig. 1 with the electronics component detached from the IV tube cassette holding the IV tube in an open state;
FiIG. 3 is a fragmentary, cut-away view of the electronics component of Fig. 1 holding peristaltic pumping elements engaging with the IV tube held by the IV tube cassette to pump fluid in a closed state;
FIG. 4 is a perspective view of a bottle connector according to one embodiment of the present invention used to connect the drug container to the IV tube held by the IV tube cassette;
FIG. 5 is a front perspective view of an alternative embodiment of the ambulatory pump with a disposable support housing receiving an IV bottle and supporting a removable electronics component and a preinstalled IV tube;
FIG. 6 is a rear perspective view of the alternative embodiment of Fig. 5 showing the electronics components removed from the support housing and the support housing receiving the IV bottle and holding the preinstalled IV tube;
FIG. 7 is a partial sectional view of the ambulatory pump of Fig. 5 showing another embodiment of a bottle connector;
FIG. 8 is a simplified perspective view of an ambulatory pump assembly as provided to a patient;
FIG. 9 is a front elevational view of a disposable drug holder for the ambulatory pump assembly of Fig. 8 with a drug container removed;
FIG. 10 is a perspective fragmentary view of an upper electronics portion of the ambulatory pump assembly of Fig. 8 with an IV tube cartridge released showing various elements thereof; and
FIG. 11 is an exploded, perspective view of a bottle connector disconnected from a right angle Luer connector used to connect a drug container to an IV tube for the ambulatory pump assembly of Fig. 8.

In describing the various embodiments of the invention which are illustrated in the drawings, specific terminology will be resorted to for the sake of clarity. However, it is not intended that the invention be limited to the specific terms so selected and it is understood that each specific term includes all technical equivalents which operate in a similar manner to accomplish a similar purpose. For example, the word "connected," "attached," or terms similar thereto are often used. They are not limited to direct connection but include connection through other elements where such connection is recognized as being equivalent by those skilled in the art.

### DETAILED DESCRIPTION OF THE PREFERRED EMBODIMENT

The various features and advantageous details of the subject matter disclosed herein are explained more fully with reference to the non-limiting embodiments described in detail in the following description.

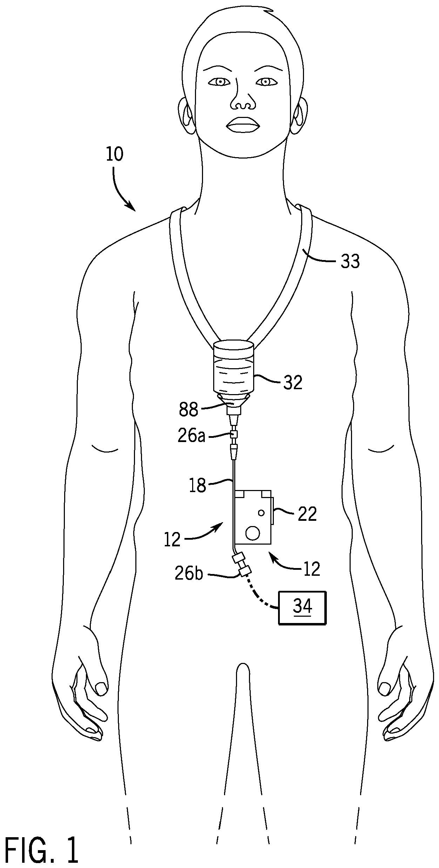

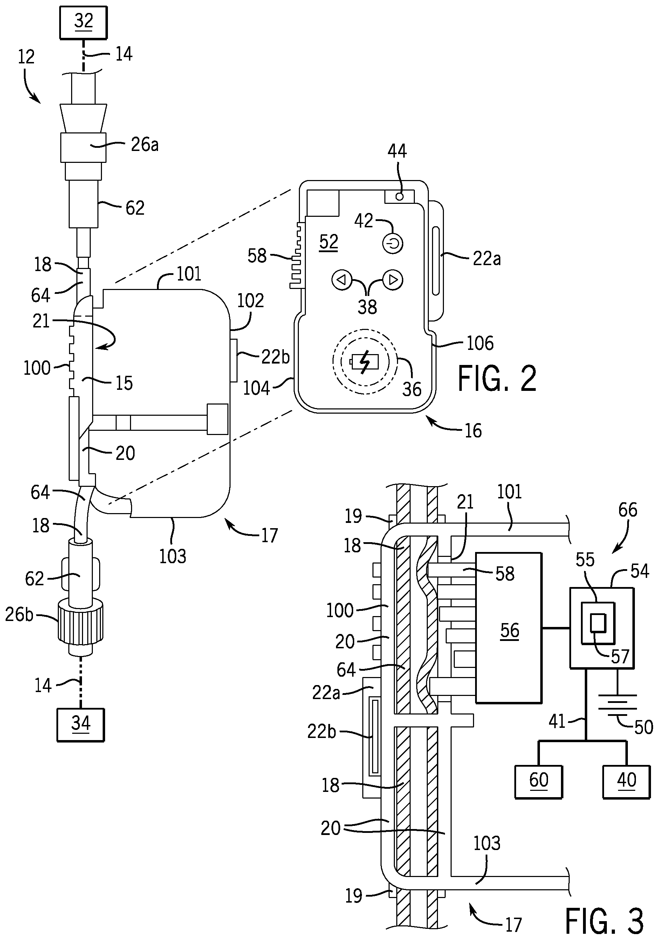

Referring to Figs. 1 and 2, a wearable pump assembly 10 per the present invention may provide for a rigid pump housing 12, for example, constructed of injection molded thermoplastic having selectively interlocking sidewall elements to open into a separate upper electronics portion 16 and a lower IV cassette portion 17 holding the IV tubing 18. The upper electronics portion 16 receives a short length of IV tubing 18 held by the lower IV cassette portion 17 to allow the peristaltic pumping elements to contact the IV tubing 18 as further described below. The upper electronics portion 16 and lower IV cassette portion 17 close together to retain the IV tubing 18 within the rigid pump housing 12 as the IV tubing 18 passes through the rigid pump housing 12 and out opposite ends of the rigid pump housing 12 along axis 14.

Referring now also to Fig. 3, the lower IV cassette portion 17 to which the IV tubing 18 is attached, may provide for retaining collars 19 within upstanding longitudinally opposed cassette end walls 101, 103, separating opposed upstanding peripheral longitudinally extending sidewalls 100, 102, and assembled around the IV tubing 18 to clamp the IV tubing 18 without crushing or closing the IV tube lumen and yet held along longitudinal guide walls 20 and axis 14 so that the lower IV cassette portion 17 is retained on the IV tubing 18 without slippage therealong. The weight of the lower IV cassette portion 17 and the upper electronics portion 16 and its contained components when attached, may be fully supported by the IV tubing 18. The longitudinal guide walls 20 may provide a cutout window 21 which permits peristaltic plunger elements 58 of the upper electronics portion 16 to access the IV tubing 18 when the upper electronics portion 16 is snapped into the lower IV cassette portion 17, as further described below.

The separate upper electronics portion 16 and lower IV cassette portion 17 may be attached by inserting a peripheral longitudinal extending sidewall 104 of the upper electronics portion 16 underneath an inwardly extending overhang 15 of the lower IV cassette portion 17 extending inwardly from the longitudinally extending sidewall 100 to restrain the longitudinal extending sidewall 104 of the upper electronics portion 16 along the longitudinally extending sidewall 100 of the lower IV cassette portion 17.

The upper electronics portion 16 and lower IV cassette portion 17 may be held in the closed, attached state against the resilience of the IV tubing 18 by sidewall latch elements 22 (as shown separated in Fig. 2) providing a temporary locking feature, for example, an outwardly extending cantilevered bar 22a of the upper electronics portion 16 with a rectangular opening flexing outwardly from longitudinally extending sidewall 106 to snap over a rectangular button 22b extending outwardly from longitudinally extending sidewall 102 of the lower IV cassette portion 17.

The ends of the IV tubing 18 extending outwardly from the lower IV cassette portion 17 may have standard Luer locks 26a and 26b allowing the ends to be connected to connectors, for example, leading from a vial, or an IV bottle, 32 elevated for gravity flow at one end by, for example, connection to a neck strap 33 worn around the neck of the patient, and to a needle set or port 34 or the like at the other end attached to a patient (as shown in Fig. 1). A central portion of the IV tubing 18, extending between each end is flexible or elastomeric to interact with plunger elements 58 of a peristaltic pump assembly 66 as will be described in more detail below. According to one aspect of the invention, the central portion of the IV tubing 18 is made from a polyvinyl chloride (PVC) peristaltic tubing.

Generally, the wearable pump assembly 10 will be both small and of light weight, for example, having a longest dimension less than 4 inches and desirably less than 3 inches and a total weight of less than 8 ounces and desirably less than 6 ounces to be easily supported by the IV tubing 18 and the Luer locks 26a and 26b without undue distortion of or damage to the IV tubing 18 or damage to the IV bottle 32 or discomfort to the patient.

Referring now to Figs. 2 and 3, the upper electronics portion 16 may include a microcontroller 54 being an electronic computer having a self-contained nonvolatile memory 55 holding an operating program 57 and necessary storage variables as will be described below. The nonvolatile memory may comprise, for example, flash memory and/or read only memory, or other similar nonvolatile memory as context requires, which may store data values to be retained even in the absence of electrical power.

The microcontroller 54 may provide control lines to a pump assembly 66 of the upper electronics portion 16 having, for example, an internal DC electric motor 56 operating through a gear system to activate the peristaltic plunger elements 58 that may press against the elastomeric central portion of the IV tubing 18 through window 21 to push fluid therethrough. As is understood in the art, generally the peristaltic plunger elements 58 extend in an undulating serpentine fashion to compress and release the tubing thereby moving fluid therethrought

The microcontroller 54 also provides various inputs and output lines communicating, for example, with a user interface on an upper face 52 of the upper electronics portion 16 of the housing 12 exposing a low battery light indicator 36 warning the user of a low battery state and that the battery needs to be recharged or replaced as discussed below. Membrane buttons 38 may allow for adjustment of the flow rate and maximum flow amount through the IV tubing 18 as will be discussed. In addition, an on/off power switch 42 allows the wearable pump assembly 10 to be turned off for storage or the like with an on state indicated by a visual indicator light 44 being lit. An optional charging port may also be provided as will be discussed below. A piezoelectric audio transducer 40 provides audible tones, for example, warning if the upper electronics portion 16 and lower IV cassette portion 17 are not fully attached or if flow rate stops or drops below a predetermined lower limit, for example, caused by kinking or occlusion in the IV tubing 18. As will be discussed, flow sensors 60 may also detect air bubbles. It is understood that the pump assembly 66 may include or omit any combination of computer controlled functions described above.

In one embodiment, upper electronics portion 16 may hold a long-life battery 50, for example, having a 10-year nominal rating such as non-rechargeable lithium thionyl chloride 3.6 volt cell, making the pump assembly 66 suitable for long-term storage and use in emergency kits or the like. Alternatively, standard lithium-ion rechargeable cells or other similar batteries may be used that may be recharged through the charging port mentioned above. The long-life battery 50 may be wirelessly recharged or AC/DC charged via charging port, or the long life battery 50 may be single use batteries that are disposed after a single use.

The battery 50 may provide power to the microcontroller 54 (through on/off power switch 42 shown in Fig. 2) controlling the motor 56 held by the upper electronics portion 16 and providing communication to the low battery light indicator 36, membrane buttons 38, and audio transducer 40 per interconnect wiring 41. The motor 56 operates through a gear system to activate peristaltic plunger elements 58 within the upper electronics portion 16 that may press against the IV tubing 18 when the upper electronics portion 16 is attached to the lower IV cassette portion 17 to push fluid therethrough.

The peristaltic plunger elements 58 may extend through the window 21 formed through longitudinal guide walls 20 toward the IV tubing 18 to press against the IV tubing 18 when the upper electronics portion 16 and lower IV cassette portion 17 are in closed position. The peristaltic plunger elements 58 extend in an undulating serpentine fashion to compress and release the tubing thereby moving fluid therethrough at a desired flow rate.

In one embodiment, the lower IV cassette portion 17 holds the IV tubing 18 which may include two end-most stiff vinyl tubing portions 62 attaching each to the Luer locks 26a and 26b and joined respectively to ends of a softer, elastomeric central tubing section 64, where the central tubing section is flexible and suitable for engaging with the peristaltic plunger elements 58 when the upper electronics portion 16 and lower IV cassette portion 17 are attached. Optionally, the central section may be made from a PVC peristaltic tubing.

This operation of the motor 56 and peristaltic plunger elements 58 may be according to a feedback loop using flow sensors 60 built into the upper electronics portion 16. The microcontroller 54 receives a desired flowrate describing a rate at which the medicament is to be delivered from the vial. According to one aspect of the invention, a user may adjust the flow rate using the membrane buttons 38 on the electronics portion 16. An indicator, such as a series of Light Emitting Diodes (LEDs), a dial, or a display unit may indicate a flow rate. The user may set the flow rate to the desired flow rate by pressing an up or down button. Optionally, the microcontroller 54 may store a series of different settings, where a desired setting may be identified by a letter, a number, or a by an indication of a drug and flow rate on the display. Pressing the membrane buttons 38 may toggle between different settings until a desired setting is selected. A pharmacist may assist a patient in setting the flow rate to a correct flow rate or may provide instructions with a vial as to the proper setting for the device.

According to another aspect of the invention, the electronics portion 16 may include selection switches internal to the housing. The selection switches may include one or more variable resistors, where the variable resistors are adjusted to provide a desired flow rate. Optionally, a series of selection switches, such as dual, in-line package (DIP) switches may be set to desired positions to set a desired flow rate. A manufacturer or a dispensing pharmacist may be trained to set the selection switches according to a specific medicament to be dispensed. The electronics portion 16 may be set once and then utilized with multiple vials of the same medication. Optionally, a patient may bring the electronics portion 16 back to the prescribing pharmacist to adjust settings on the electronics portion 16 for use with a different medicament.

Each flow sensor 60 generates a feedback signal corresponding to a measured flow rate for the medicament past the corresponding flow sensor. The feedback signals are provided to the microcontroller 54. The microcontroller may compare the measured flow rate to the desired flow rate and either speed up or slow down the peristaltic pump assembly 66 to achieve the desired flow rate.

Under a range of different pressures of IV fluid in the IV tubing 18, for example, the motor 56 may adjust the speed of the peristaltic plunger elements 58 to provide a steady flow rate at a set point of the feedback loop according to the measured flow through the IV tubing 18 by the flow sensors 60. For example, upstream and downstream pressure sensors can be used to ensure proper operation of the pump by detecting abnormal pressures. Generally, the flow sensors 60 may provide a spring-loaded plunger that presses into the outer wall of the IV tubing 18 to sense pressure. It is understood that in certain embodiments, the sensors may be omitted to provide simplified operation.

Referring again to Fig. 1, while the housing 12 may be fully self-supporting on the IV tubing 18, the IV bottle 32 may be held by neck strap 33 attached the IV bottle 32, e.g., wrapped around and secured to the bottom of the IV bottle 32, while the IV bottle 32 is suspended in an inverted position and hung from the neck of the patient to facilitate gravity flow. Self-supporting in this context means that the pump housing 12 can be indefinitely supported on the IV tubing 18, for example, the latter suspended vertically for example from IV bottle 32, without undue distention of the IV line or damage thereto. Optionally, other patient support members may be provided, where a patient support member is a device used to support the pump assembly 10 on the patient. Suitable patient support members include, for example, a belt, an adjustable strap, a clip, or the like, which allow the pump assembly 10 to be "worn" by the patient as the patient moves about.

In use, the lower IV cassette portion 17 is pre-installed with the IV tubing 18, optionally with a softer central tubing section 64 made from silicone rubber or from a PVC peristaltic material, and further attached to connectors via the Luer locks 26a and 26b at the ends of the IV tubing 18. The preassembled lower IV cassette portion 17 may be prepared by the pharmacist and shipped to the patient with the prefilled drug container or IV bottle 32.

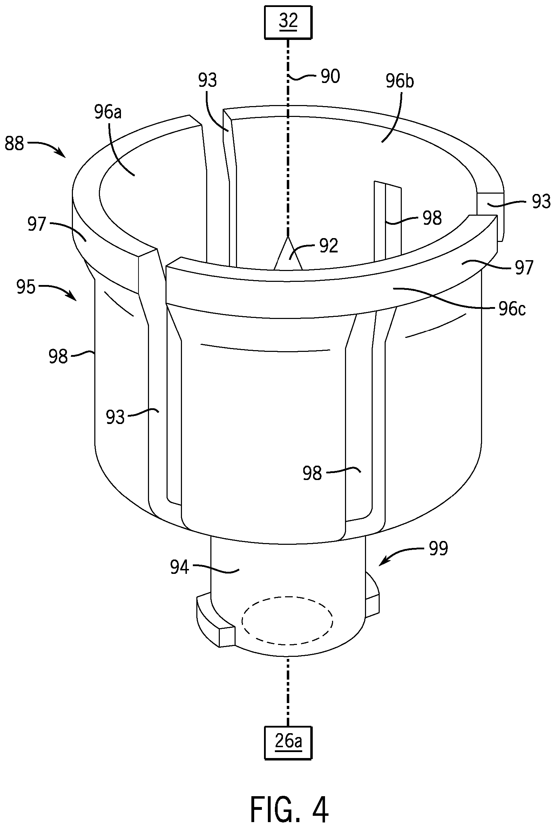

Referring briefly to Fig. 4, a bottle connector 88 may be pre-attached to the Luer lock 26a to allow the patient to connect the IV bottle 32 to the Luer lock 26a and permit flow from the IV bottle 32 through the Luer lock 26a to the IV tubing 18.

The bottle connector 88 may be a cylindrical cap surrounding a central hollow needle 92 extending along a needle axis 90 that attaches over the cap of the IV bottle 32, for example, providing three axial slots 93 extending along the needle axis 90 and separating the top portion 95 into cantilevered sections 96a, 96b, 96c that are flexible outwardly and inwardly toward the needle axis 90 at its top rim 97. Additional axial slots 98 extending along the needle axis 90 may be formed within the top portion 95 below the top rim 97 to provide additional flex.

The cantilevered sections 96a, 96b, 96c are expanded over the cap of the drug container and snapped onto the cap of the IV bottle 32 to further puncture a seal of the cap of the IV bottle 32 via the hollow needle 92 extending through the seal to allow liquid medicament to be withdrawn from the volume of the IV bottle 32. The bottle connector 88 may be vented to remove bubbles.

The bottom portion 99 of the bottle connector 88 supports a tube connector 94 attached to the Luer lock to permit fluid inside the IV bottle 32 to flow through the hollow needle 92 through the bottle connector 88 to provide a leak proof communication between the hollow needle 92, bottle connector 88, and Luer lock 26a. The bottle connector 88 and/or Luer lock 26b may include a stop valve to stop flow before desired drug delivery. The IV bottle 32 may be hung from the patient's neck using the neck strap 33 attached to the inverted the IV bottle 32 during drug delivery.

Referring again to Fig. 2 and 3, the preassembled lower IV cassette portion 17 is received by the patient and the upper electronics portion 16 snapped onto the lower IV cassette portion 17 to form the closed pump housing 12. Thus, the peristaltic plunger elements 58, extending outwardly from the longitudinally extending sidewall 104 opposite the peripheral longitudinally extending sidewall 106, are inserted underneath the inwardly extending overhang 15 of the lower IV cassette portion 17 to allow the peristaltic plunger elements 58 to extend through the window 21 of the longitudinal guide walls 20. The latch element 22b of the peripheral longitudinally extending sidewall 106 and the latch element 22a of the upstanding peripheral longitudinally extending sidewall 102 are snapped together to temporarily lock the upper electronics portion 16 and lower IV cassette portion 17 together.

The pump assembly 66 may be switched on, triggering a self-check confirming operation of the peristaltic plunger elements 58, for example, by monitoring excess current or a limit switched as the motor moves the peristaltic plunger elements 58. A flow rate may be preloaded and the pump assembly 66 may begin operation immediately, for example, in emergency applications. Otherwise, the membrane buttons 38 may be used to set a desired flow rate.

It will be appreciated that the flow rate may be implemented by a mobile device such as a separate control computer, smart phone or tablet (henceforth smart device) securely linked with the microcontroller 54, for example, through a near field communication device such as Bluetooth or Wi-Fi connection so that its own keyboard, display, and sensing elements may be used. As used herein, near field communication refers to a wireless technology typically operating at a range of zero to five centimeters and not operating at distances of greater than approximately one meter. In this situation, a single mobile device such as an iPhone or Android operating system phone is linked to the microcontroller 54 of the upper electronics portion 16 in a secure manner.

During infusion, flow is monitored by the flow sensors 60 and controlled by the controller 54. Warning tones are produced if flow is blocked or obstructed or an air bubble is detected (upon which the pinch valve closes).

If a total desired dose amount has been entered by the user, at the conclusion of that dose delivery (obtained by integrating the flow rate) the peristaltic plunger elements 58 are stopped and a signal is provided to the user.

At the conclusion of the infusion, the lower IV cassette portion 17 with the preinstalled IV tubing 18 connected to the IV bottle 32 may be removed from the upper electronics portion 16 and replaced with a new lower IV cassette portion 17 that has sterile properties. The previously used lower IV cassette portion 17 with preinstalled IV tubing 18 connected to the IV bottle 32 disposed. The microcontroller 54 may track the battery reserve, for example, by counting the number of motor cycles, battery age, recharges, etc. to ensure ample capacity exists for the next infusion. Thus, a single electronics portion 16 may be reused with multiple IV cassette portions 17, thereby, reducing the overall cost of a single dosage.

In at least some embodiments, the microcontroller 54 may check the battery reserve prior to every new drug delivery operation or motor 56 and flow sensors 60 operation to determine if there is sufficient battery reserve or battery life remaining for a typical drug delivery. The controller may check for adequate remaining battery energy for the drug delivery using an internal time-to-ampere-hour conversions based on the current drain of the microcontroller 54, motor 56 and flow sensor 60 and compare that against the estimated total ampere-hours of the battery 50. If the battery reserve is less than what would be needed, the microcontroller 54 may indicate to the user that the battery 50 should be charged or replaced prior to operation. The microcontroller 54 may not permit operation of the motor 56 and flow sensors 60 until the battery 50 is charged to a sufficient level to prevent unwanted interruptions during delivery.

In at least some embodiments, the number of uses of the pump assembly 66 may be counted, for example, determined by accumulating the number of IV tubing replacements, motor cycles, battery life, recharges, and other lifespan tracking methods, in order to alert the user or automatically shut off when the pump assembly 66 has reached its intended service life limits, should no longer be used, and should be disposed. Therefore, it is understood that the pump assembly 66 may be intended as a disposable use device with limited service life.

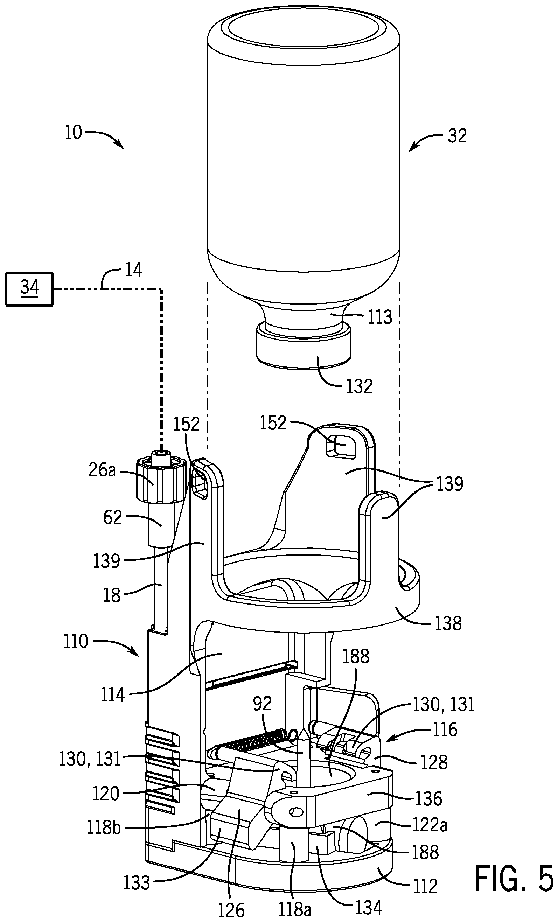

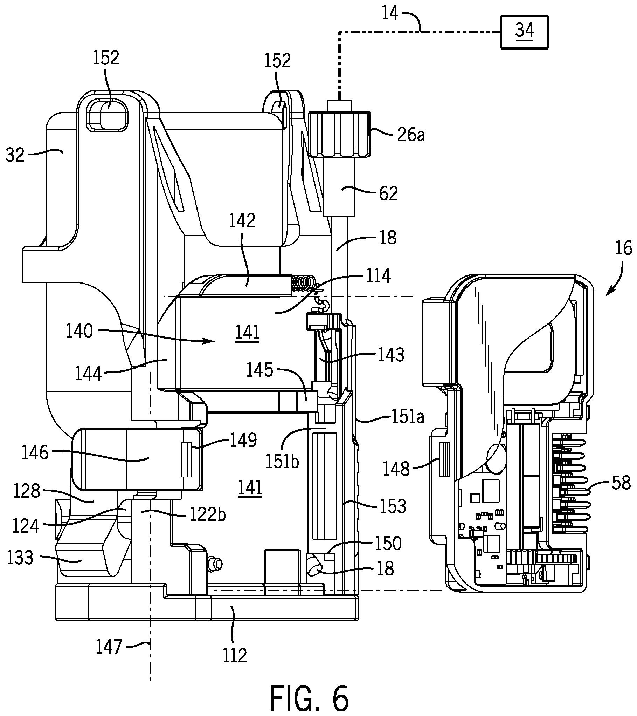

In an alternative embodiment shown in Figs. 5 and 6, the wearable pump assembly 10 may include a disposable support housing 110 replacing the lower IV cassette portion 17 of Figs. 1 through 3 and supporting the preinstalled IV tubing 18 and the removable upper electronics portion 16 so that they are in close engagement with a vial, or IV bottle, 32 providing liquid medicament, all of which are supported on the support housing 110. The upper electronics portion 16 removably attaches to the support housing 110 to align the peristaltic plunger elements 58 of the upper electronics portion 16 against the preinstalled IV tubing 18 as the IV tubing 18 passes through the support housing 110 along axis 14.

Referring specifically to Fig. 5, the support housing 110 may be a generally L-shaped frame having a horizontal platform 112 extending horizontally to receive an inverted IV bottle 32 thereon and a vertical wall 114 extending upwardly from a rear of the horizontal platform 112 to provide a rear support wall receiving the upper electronics portion 16 thereagainst. The IV bottle 32 and the upper electronics portion 16 are in close alignment when supported by the support housing 110 so as to allow fluid to flow from the IV bottle 32 through the preinstalled IV tubing 18 and through the support housing 110 to be further pumped to the needle set or port 34 or the like at the other end attached to a patient through a needle or a port (as shown in Fig. 1 and as described above).

The horizontal platform 112 may include a locking assembly 116 to lock the neck 113 of the IV bottle 32 to the horizontal platform 112. The locking assembly 116 may include a left pair of front and back vertical pins 118a, 118b supporting a horizontal pin 120 at their top ends extending along a left side of the horizontal platform 112. The locking assembly 116 may further include a right pair of front and back vertical pins 122a, 122b supporting a horizontal bar 124 at their top ends extending along a right side of the horizontal platform 112.

The horizontal pins 120, 124 receive hinged latches 126, 128, respectively, which pivot about the horizontal pins 120, 124 with an inward end 130 extending downward to engage with the neck 113 of the vial, or IV bottle, 32 and extending upward to disengage with the neck 113 of the vial, or IV bottle, 32. Specifically, the engagement of the hinged latches 126, 128 with the neck 113 of the vial 32 causes hooks 131 at the inward end 130 of the hinged latches 126, 128 to hook onto the sterile cap 132 on the neck 113 of the vial 32 and exert force downward on the sterile cap 132 against a preinstalled bottle connector 188 supported on the horizontal platform 112. As the hinged latches 126, 128 are pressed into a locked condition, the force applied to the neck 113 of the vial 32 causes the vial 32 to engage the central needle 92 and to force the central needle through a seal or membrane present over the mouth of the vial 32 and into an interior volume 35 of the vial. The hinged latches 126, 128 also secure the vial 32 against the bottle connector 188 within the disposable support housing 110.

An opposite end 133 of the hinged latches 126, 128 extend outwardly to allow a user to press inwardly and downwardly on the opposite end 133 to release the hooks 131 from engagement. In the disengaged position, the hooks 131 are released from the sterile cap 132 on the neck 113 of the IV bottle 32 and the IV bottle 32 may be removed from the horizontal platform 112. A spring (not shown) may bias the hinged latches 126, 128 in the engaged position to make the engaged position the default position.

According to one embodiment of the invention, the bottle connector 188 may be as similarly described above with respect to the bottle connector 88 illustrated in Fig. 4, but the hollow needle 92 may extend above the top rim 97 to more easily engage the fluid and the tube connector 94 connecting the IV tubing 18 may be a L-shaped, T-shaped, or 90-degree connector extending horizontally along the horizontal platform 112.

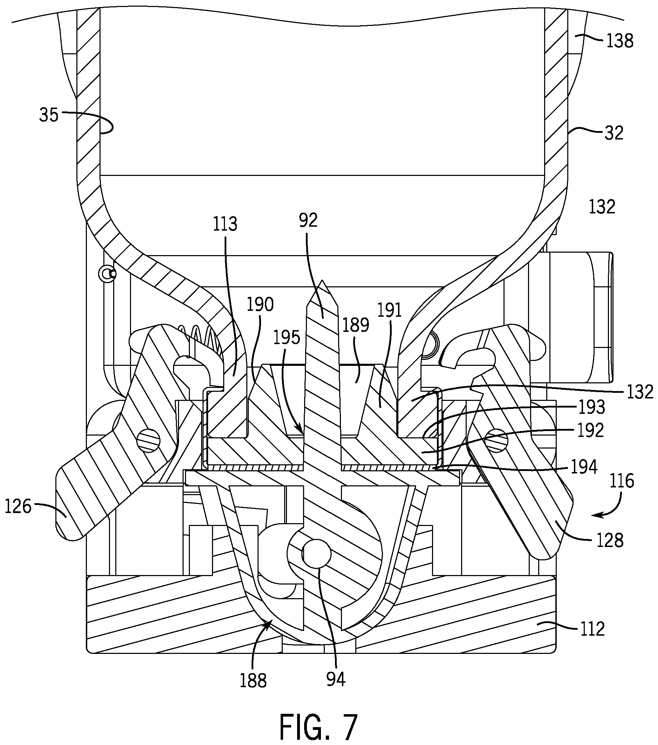

According to another embodiment of the invention, the bottle connector 188 may include a compliant seat 193 against which the vial 32 is secured. With reference to Fig. 7, the bottle connector 188 includes a first compliant member 189 and a second compliant member 192. The first compliant member 189 has an upper portion 190 with a tapered outer periphery configured to fit within the neck 113 of the vial 32 and to guide the vial onto the first compliant member 189. The first compliant member 189 includes a lower portion 191 with an annular outer periphery configured to fit within the neck 13 of the vial 32 and to establish a seal between the first compliant member 189 and an interior periphery of the neck 13 of the vial 32. It is contemplated that the diameter of the lower portion 191 may be slightly greater than a diameter of the inner periphery of the neck 113 of the vial 32. As the hinged latches 126, 128 draw the vial 32 onto the first compliant member 189, the lower portion 191 may compress to fit within the inner periphery of the neck 113 of the vial 32 and provide a more secure seal therebetween. The second compliant member 192 is generally annual with an outer periphery greater than the outer periphery of the first compliant member 189. An upper surface 193 of the second compliant member 192 defines the seat against which the vial 32 is secured by the latches 126, 128.

Each of the first and second compliant members 189, 192 include an opening through the center through which the pointed end of the hollow needle 92 extends. The lower end of the hollow needle 92 extends downward past the first and second compliant members 189, 192 to a tube connector 94, defining a fluid communication path between the hollow needle and the tube connector 94. A first end of the IV tube 18 is connected to the tube connector 94 to create a fluid flow path from inside the vial 32, through the hollow needle 92, through the tube connector 94, and to the preinstalled IV tubing 18 further engaging with the upper electronics portion 16 as will be described below. Upstanding lateral retaining walls 134 may further flank to retain the bottle connector 188 on the horizontal platform 112.

A lower retaining ring 136 extending forwardly from the vertical wall 114 to provide an upwardly exposed circular opening extends around the top rim 97 of the bottle connector 188 and the narrower portion of the inverted IV bottle 32 to help retain the IV bottle 32 on the horizontal platform 112 in the inverted position.

An upper retaining ring 138 extending forwardly from the vertical wall 114 to provide an upwardly exposed circular opening extends around the broader portion of the inverted IV bottle 32 to retain the IV bottle 32 on the horizontal platform 112 and along the vertical wall 114. The upper retaining ring 138 may support upwardly extending retaining fingers 139 which extend upward along the body of the IV bottle 32.

The opening of the upper retaining ring 138 is generally larger than the lower retaining ring 136 to circumscribe a circumference of a body of the lower portion of the IV bottle 32 whereas the lower retaining ring 136 circumscribe a circumference of the narrower neck 113 of the upper portion of the IV bottle 32.

Referring to Fig. 6, the vertical wall 114 supports attachment of the upper electronics portion 16 thus allowing the preinstalled IV tubing 18 to extend from the bottle connector 188 and along the upper electronics portion 16 to permit the peristaltic plunger elements 58 to engage with the preinstalled IV tubing 18. A rear surface 141 of the vertical wall 114 may include a rectangular depression 140 receiving the electronics portion 16 against the vertical wall 114 and further being restrained by upper retaining wall 142, lateral left and right retaining walls 143, 144, and the rear edge of the horizontal platform 112 to keep the upper electronics portion 16 supported against the vertical wall 114.

A hook 145 on the left side of the rectangular depression 140 may hook onto the left sidewall of the upper electronics portion 16 thus locking the left side to the vertical wall 114.

A spring bias latch 146 may extend along the right side of the vertical wall 114 to pivot inwardly about a hinge axis 147 extending along the right side to allow a tab 149 with a receiving hole to pivot inwardly to engage a corresponding button 148 along a right sidewall of the upper electronics portion 16 thus locking the upper electronics portion 16 to the vertical wall 114 and to pivot outwardly to disengage the corresponding button 148 of the right sidewall of the upper electronics portion 16. The spring bias latch 146 may be manually pressed to disengage the corresponding button 148 from the tab 149. A spring (not shown) may bias the spring bias latch 146 in the engaged position to make the engaged position the default position.

The vertical wall 114 may support a small cutout hole 150 therethrough permitting the preinstalled IV tubing 18 to extend from the bottle connector 188 at the front side of the vertical wall 114 to the upper electronics portion 16 on an opposite side of the vertical wall 114.

Additional guides or guide walls 151 extending rearwardly from the rear surface 141 of the vertical wall 114 further align and retain the preinstalled IV tubing 18 along the peristaltic plunger elements 58 of the upper electronics portion 16. These outer and inner guide walls 151a, 151b may extend generally along the left side of the rear surface 141 and flank the outer and inner sides of the preinstalled IV tubing 18 to allow the tubing to extend vertically upward along the vertical wall 114 to vinyl tubing portions 62 attaching each to the Luer lock 26a as previously taught.

The peristaltic plunger elements 58 may extend outwardly from the upper electronics portion 16 and thus the preinstalled IV tubing 18 is pressed toward the outer guide wall 151a of the vertical wall 114 by the actuating elements. The peristaltic plunger elements 58 may extend through a cutout window 153 of the inner guide wall 151b to press toward the outer guide wall 151a. Accordingly, the peristaltic plunger elements 58 extend outwardly from the upper electronics portion 16 to engage the preinstalled IV tubing 18 of the support housing 110.

The support housing 110 may be suspended on the patient's body by the neck strap 33 which is threaded through holes 152, for example, within the upwardly extending retaining fingers 139. Thus, the support housing 110 is self-contained and the IV bottle 32 and upper electronics portion 16 are fully self-supported by the support housing 110. Optionally, the holes 152 may be arranged in a generally vertical arrangement, allowing a belt or strap to be inserted, where the belt or strap is used to secure the support housing 110 to the patient. According to still another option, a clip may be provided to clip the support housing 110 to the patient.

Referring again to Figs. 5 and 6, in use, the patient may connect the IV bottle 32 to the support housing 110 by inserting the IV bottle 32 downwardly through the upper retaining ring 138 and the lower retaining ring 136 to attach the IV bottle 32 to the bottle connector 188, and the hinged latches 126, 128 engage with the neck 113 of the IV bottle 32 to lock the IV bottle 32 to the bottle connector 188 and the horizontal platform 112.

When the IV bottle 32 is attached to the bottle connector 188, the hollow needle 92 extends through the seal of the sterile cap 132 of the IV bottle 32 to allow liquid medicament to be withdrawn from the volume of the IV bottle 32. The bottle connector 188 then communicates fluid to the preinstalled IV tubing 18 which extends from the bottle connector 188 to the upper electronics portion 16.

Further, the upper electronics portion 16 is attached by the user to the vertical wall 114 by aligning the upper electronics portion 16 to the rectangular depression 140 on the rear surface 141 of the vertical wall 114 and then latching the hook 145 onto the upper electronics portion 16 and spring bias latch 146 into the corresponding tab 148 along the right sidewall of the upper electronics portion 16 to lock the upper electronics portion 16 to the vertical wall 114 thus allowing the peristaltic plunger elements 58 to engage with the preinstalled IV tubing 18 for peristaltic pumping.

In order to deliver the medicament to the patient, the controller 54 regulates operation of the plunger elements 58 in the pump assembly 66 to engage the IV tube 18 within the pump assembly 10. The pump assembly 66 works in cooperation with gravity to draw the medicament from the vial 32. With the vial 32 inserted upside down into the support housing 110, the medicament is present at the neck 113 of the vial by the hollow needle 92. The plunger elements 58 compress and release the IV tube 18 in a sequential manner to draw the fluid from the vial 32. Fluid within the tube is urged forward by the sequential compression of the IV tube 18 by the plunger elements 58. As plunger elements 58 release the IV tube 18, a vacuum is created within the tube to draw additional fluid from the vial 32 or IV container to which the IV tube 18 is connected. The vacuum draws the medicament through the hollow needle 92 and the tube connector 94 within the bottle connector 188. The first end of the IV tube 18 is connected to the tube connector 94, and the plunger elements 58 force the medicament through the IV tube 18 and out the second end of the IV tube 18. A second connector is present at the second end of the IV tube 18 to connect to further IV tubing between the pump assembly 10 and a port or needle which delivers the medicament to the patient. According to one aspect of the invention, each of the first and second connectors on the first and second ends of the IV tube 18 are Luer lock 26a, 26b connectors.

In order to improve the pumping process, it may be desirable to introduce air into the vial 32 above the medicament. As the medicament is drawn from the vial 32, a vacuum would be generated within the vial 32 above medicament. The vacuum in the vial 32 may counter the vacuum generated by the pump assembly 66, making the pumping process less efficient. An opening 195 may be provided in the bottle connector 188 at a position above the point where the medicament exits the bottle connector. According to the illustrated embodiment, the opening around the hollow needle 92 is utilized as an opening 195 in which to introduce air from outside the vial. A hydrophobic filter 194 is provided between the bottom of the second compliant member 192 and a seat of the bottle connector 188. The hydrophobic filter 194 permits air to pass through while preventing water from passing. As a result, the vacuum created in the upper portion of the vial 32 as medicament is drawn from the vial will draw air from the ambient environment outside of the vial into the interior volume 35 of the vial 32. Introduction of the air from outside the vial 32 into the portion of the interior volume 35 vacated by the medicament equalizes the pressure on the medicament and allows the peristaltic pump assembly 66 to continue drawing additional medicament from the vial 32.

At the conclusion of the infusion, the upper electronics portion 16 may be removed from the support housing 110 to be reused with another infusion. The IV bottle 32 and the support housing 110 may be replaced with a new support housing 110 with sterile properties, and a new IV bottle 32 loaded into the new support housing 110 for the next infusion. The IV bottle 32 and the support housing 110 are thus disposable for reduced operating costs.

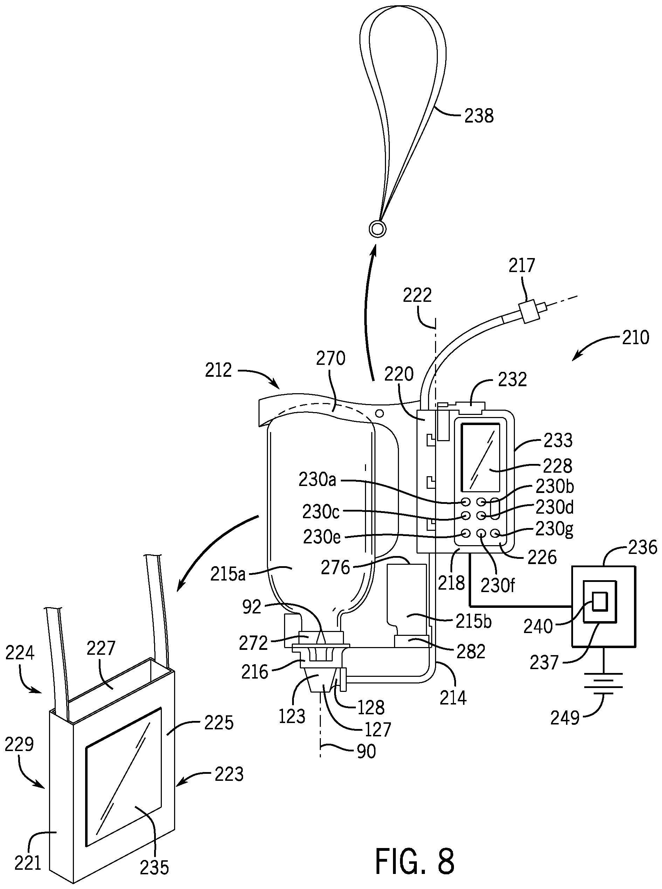

Turning next to Figs. 8-11, another embodiment of the invention is illustrated. An ambulatory pump 210 according to the illustrated embodiment may operate in conjunction with a disposable housing 212 holding an IV tube 214. The disposable housing 212 may carry a drug container 215, for example, an IV bottle or vial of the type used to hold IV solutions, and the IV tube 214 provides a flexible tube allowing the flow of medicament from the drug container 215, through a bottle connector 216, and an IV tube connector 217 that may further communicate with a traditional IV line 213 connected to a patient 219 through a needle or port or the like. The IV tube 214 may include a bubble filter for removing included air bubbles, limiting the need for air bubble sensing.

The ambulatory pump 210 provides a two-part housing having an upper electronics portion 218 that may attach to an IV tube cartridge 220 to receive the IV tube 214 therebetween along a longitudinal axis 222 being generally the longest dimension of the housing of the ambulatory pump 210. As so received, the ambulatory pump 210 may pump liquid through the IV tube 214 by peristaltic action.

In one embodiment, the ambulatory pump 210 is constructed to weigh less than a half pound and preferably less than 1.5 inches by 2 inches by 5 inches so as to be easily carried by the patient 219, for example, in a compartment 227 of a pouch 224 also sized to receive the disposable housing 212.

The ambulatory pump 210 may be held within the pouch 224 with longitudinal sidewalls 221 and 223, respectively, of the compartment 227 of the pouch 224 closely cradling the corresponding longitudinal sidewalls of the ambulatory pump 210 and the disposable housing 212, front and rear walls 225 and 229, respectively, of the compartment 227 of the pouch 224 closely cradling the corresponding front and rear opposed sidewalls of the ambulatory pump 210 and the disposable housing 212, and the front wall 225 of the pouch 224 having a clear screen 235 providing a window that allows the user to view a liquid crystal type display 228 of the ambulatory pump 210 and to, optionally, press membrane switch pushbuttons 230 of a user interface 226 of the ambulatory pump 210 through the clear screen 235.

Alternatively, the ambulatory pump 210 may be attached to a strap 238, for example, by a keyring or clip attached to a hole at the top end of the disposable housing 212, allowing the ambulatory pump 210 to be hung or suspended from the patient 219. The strap 238 may be worn around the neck of the patient 219 to allow the ambulatory pump 210 to hang from the patient's neck. Thus, drug container 215 is held in an inverted position permitting gravity flow.

In one embodiment, the housing of the upper electronics portion 218 may present on its front wall 233 the user interface 226 comprising, for example, the liquid crystal type display 228 for displaying symbols and alphanumeric characters under computer control. The user interface 226 also provides multiple membrane switch pushbuttons 230 that may be activated by a user. Generally, the pushbuttons include a limited number of controls including, in one embodiment, run and stop pushbuttons 230a and 230b, respectively, that will stop and start operation of the pump; a rate pushbutton 230c allows setting of the maximum pumping rate of the ambulatory pump 210 in milliliters per hour by cycling through menu standard rates with each push. The pushbuttons also include a "volume to be infused" pushbutton 230d allowing user control of the maximum volume to be infused during a treatment protocol, also by cycling through standard settings with each push, as well as an information pushbutton 230e allowing the display of detailed information about the pump including remaining pump life. Pushbutton 230f allows the unit to be turned on and off to conserve power. A bolus pushbutton 230g allows short operation of the pump to deliver medicament in fixed patient-controlled bolus quantities. It is understood that the user interface 226 of the upper electronics portion 218 may include or omit the liquid crystal type display 228 and may include or not include any combination of computer controls described above with respect to pushbuttons 230a, 230b, 230c, 230d, 230e, 230f, 230g.

The ambulatory pump 210 may include a microcontroller 236 being an electronic computer having a self-contained nonvolatile memory 237 holding an operating program 240 and necessary storage variables as will be described below. The nonvolatile memory may comprise, for example, flash memory and/or read only memory, or other similar nonvolatile memory as context requires, which may store data values to be retained even in the absence of electrical power.

The microcontroller 236 also provides various inputs and output lines communicating, for example, with the display 228 for providing display information thereon and various pushbuttons 230 for receiving data related to their activation by user. In addition, the microcontroller 236 may provide control lines to a pump assembly having, for example, an internal DC electric motor (not shown) operating through a gear system to activate peristaltic plunger elements 244 that may press against the contained IV tube 214 to push fluid therethrough. As is understood in the art, generally the peristaltic plunger elements 244 extend in an undulating serpentine fashion to compress and release the tubing thereby moving fluid therethrough.

The microcontroller 236 may also communicate electrically with various sensors. For example, upstream and downstream pressure sensors 246 and 248 can be used to ensure proper operation of the pump by detecting abnormal pressures. Generally, each of the pressure sensors 246 and 248 may provide a spring-loaded plunger that presses into the outer wall of the IV tube 214 to sense pressure. It is understood that in certain embodiments, the sensors may be omitted to provide simplified operation.

All electrical components in the upper electronics portion 218 may be supplied with power by a contained storage battery 249 that may provide its power directly or through standard power processing circuits such as regulators and the like. The contained storage battery 249 may be wirelessly recharged or AC/DC charged via micro-USB port, or the contained storage battery 249 may be single use batteries that are disposed after a single use.

It will be appreciated that the user interface 226 elements of display 228 may be implemented by a mobile device such as a separate control computer, smart phone or tablet (henceforth smart device) securely linked with the microcontroller 236, for example, through a near field communication device such as Bluetooth or Wi-Fi connection so that its own keyboard, display, and sensing elements may be used. As used herein, near field communication refers to a wireless technology operating at a distance of four centimeters or less and not operating at distances of greater than approximately one meter. In this situation, a single mobile device such as an iPhone or Android operating system phone is linked to the microcontroller 236 of the upper electronics portion 218 in a secure manner.

The front wall 233 provides for a release button or lever 232 to remove the IV tube cartridge 220 from attachment.

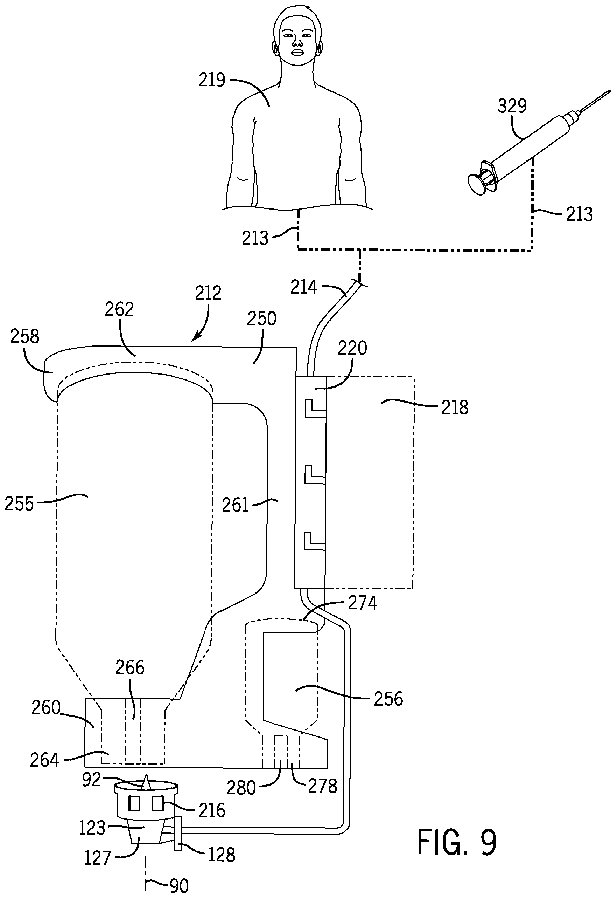

Referring also to Fig. 9, the ambulatory pump 210 provides a disposable housing 212 carrying the drug container 215 intended for disposal after use with a single patient 219. The disposable housing 212 includes a rigid frame 250 having a first and second half 252a and 252b joined to close in a closed state along a separation plane 254. In one embodiment, the rigid frame 250 may be constructed of medical grade plastic polymers such as polyethylene, poly propylene, PMMA, PVC, polyamide, ABS or polycarbonate, for example, having a thickness from 0.01 inches to 0.1 inches. The rigid frame 250 may be transparent to allow the user to more easily see the drug containers 215.

Each of the first and second halves 252a and 252b may form circular cut outs to define a large drug container volume 255 and a small drug container volume 256 within the disposable housing 212 between the first and second halves 252a and 252b. The rigid frame 250 generally has horizontally extending restraining walls extending along the separation plane 254 defined by an upper arm 258 and a lower arm 260 joined by a vertically extending restraining wall extending perpendicular to the upper arm 258 and lower arm 260 defined by bridge member 261.

Depressions may be formed within the retaining walls forming the upper arm 258 and the lower arm 260, and surrounding the large drug container volume 255, respectively, to abut an outer periphery of the drug container 215a held within the large drug container volume 255 to restrain movement. For this purpose, the first and second halves 252a and 252b are joined to form the upper arm 258 holding a circular depression 262 (extending away from the lower arm 260) receiving a circular bottom 270 of the drug container 215a and the lower arm 260 holding a circular depression 264 (extending away from the upper arm 258) surrounding a port 266 receiving a circular top 272 of the drug container 215a, for example, a cylindrical neck and cap of the IV bottle. The port 266 provides an opening that allows the bottle connector 216 to be connected to the neck of the IV bottle and extend therethrough. In this respect, the drug container 215a may be in an inverted position to assist with the flow of liquid medicament outward from the bottle through the bottle connector 216.

Similarly, each of the first and second halves 252a and 252b may form a small drug container volume 256 within the disposable housing 212, and specifically formed within the bridge member 261, to abut an outer periphery of the drug container 215b held within the small drug container volume 256 to restrain movement. For this purpose, the first and second halves 252a and 252b are joined to define the small drug container volume 256 with an upper circular depression 274 (extending away from the lower arm 260) receiving a circular bottom 276 of the drug container 215b and a lower circular depression 278 (extending away from the upper arm 258) surrounding a port 280 receiving a circular top 282 of the drug container 215b, for example, a cylindrical neck and cap of the IV bottle. The port 280 provides an opening that allows the bottle connector 216 to be connected to the neck of the bottle and extend therethrough. In this respect, the drug container 215b may be in an inverted position to assist with the flow of liquid medicament outward from the bottle through the bottle connector 216.

At the time of manufacture, the drug container 215 may be filled by a pharmacist and installed within the disposable housing 212 by installing the drug containers 215a and 215b into the large drug container volume 255 and small drug container volume 256, respectively, and joining the first and second halves 252a and 252b. It is understood that the disposable housing 212 may be installed with both or either of the drug containers 215a and 215b in a manner which permits the intended drug therapy to be delivered. For example, the drug therapy may require that the drug containers 215a and 215b be delivered separately, simultaneously or sequentially.

The patient 219 may receive the pre-filled drug container 215 pre-installed within the disposable housing 212 from the pharmacist and may be able to attach the bottle connector 216 to the pre-filled drug container 215 and attach the upper electronics portion 218 to the IV tube cartridge 220 on their own in the home environment as described below.

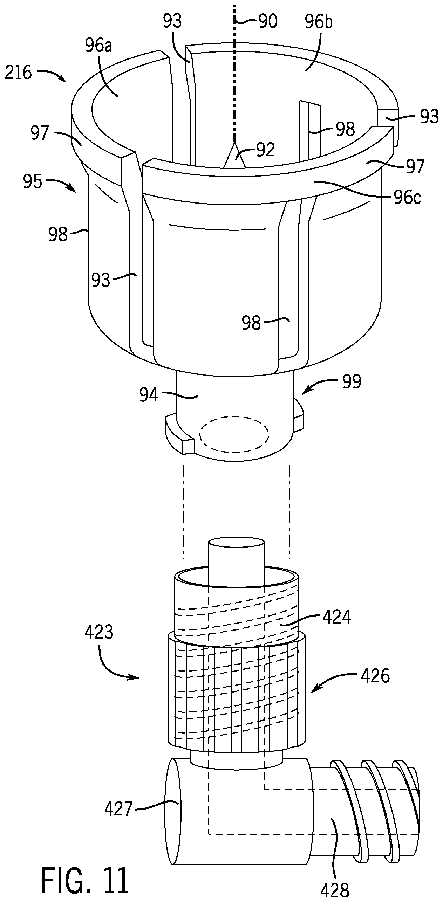

Referring also to Fig. 11, the bottle connector 216 may be connected to the drug container 215 to fluidly communicate liquid from the drug container 215 through the IV tube 214. The bottle connector 216 may be a cylindrical cap surrounding a central hollow needle 92 extending along a needle axis 90 that attaches over the cap of the drug container 215, for example, providing three axial slots 93 extending along the needle axis 90 and separating the top portion 95 into cantilevered sections 96a, 96b, 96c that are flexible outwardly and inwardly toward the needle axis 90 at its top rim 97. Additional axial slots 98 extending parallel to the needle axis 90 may be formed within the top portion 95 below the top rim 97 to provide additional flex. The cantilevered sections 96a, 96b, 96cexpand over the cap of the drug container and snap onto the cap of the drug container 215 to further puncture a seal of the cap of the drug container 215 by the hollow needle 92 extending through the seal to allow liquid medicament to be withdrawn from the volume of the drug container 215. The bottle connector 216 may be vented to remove bubbles. The bottom portion 99 of the bottle connector 216 supports a tube connector 94 to permit fluid inside the drug container 215 to flow through the hollow needle 92 through the bottle connector 216 to provide a leak proof communication between the hollow needle 92 and the bottle connector 216.

The bottle connector 216 may be further connected to a right angle Luer connector 423 providing a Luer lock 424 on a top portion 426 attachable to the tube connector 94 of the bottle connector 216 and joined by a right angle joint 427 joining the top portion 426 to a tube connector 428 extending perpendicular to the needle axis 90 to allow flow through the right angle Luer connector 423 to the IV tube 214. The tube connector 428 connects to the IV tube 214 to permit the IV tube 214 to extend perpendicular to the needle axis 90 minimizing kinking of the IV tube 214 when the disposable housing 212 is held within the pouch 224 or placed on a flat surface, thus minimizing unwanted occlusions.

The bottle connector 216 and right angle Luer connector 423 may be attached to either of the drug containers 215a and 215b or two bottle connectors 216 and right angle Luer connectors 423 may be attached to both drug containers 215a and 215b and the respective separate IV tubes 214 joined by a Y-connector to a single IV tube 214.

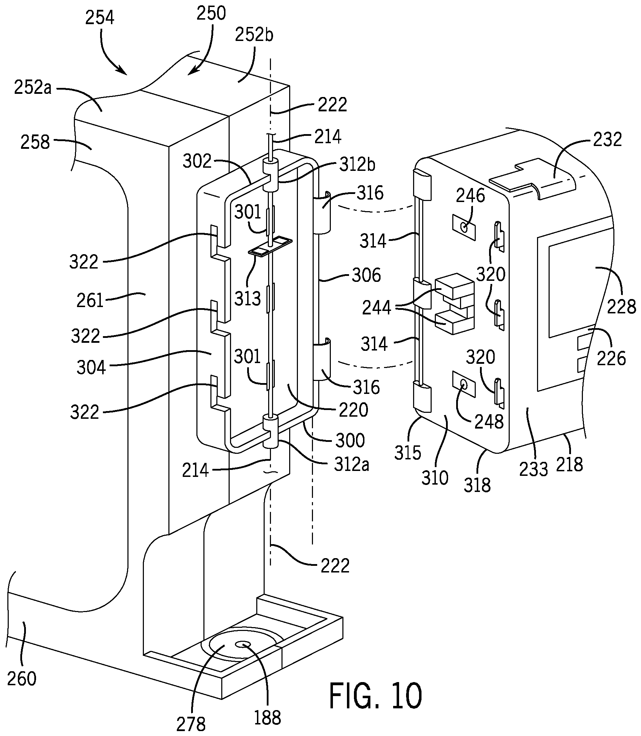

Referring to Fig. 10, the IV tube 214 is further guided to the IV tube cartridge 220 coupled to the disposable housing 212, for example, attached to the exterior surface of the bridge member 261. Guides may be used to restrain the IV tube 214 to the disposable housing 212 between the bottle connector 216 and the IV tube cartridge 220. The IV tube cartridge 220 provides a shallow tray having upstanding peripheral longitudinally-opposed end walls 300 and 302, these walls separating the opposed upstanding peripheral longitudinally-extending sidewalls 304 and 306.

The bottom wall 310 of the upper electronics portion 218 may hold peristaltic plunger elements 244 and the downwardly extending operators of pressure sensors 246 and 248 aligning with the IV tube 214 of the IV tube cartridge 220, the IV tube 214 held by guides 301 of the IV tube cartridge 220 which form notches to align and retain the IV tube 214 longitudinally within the tray. The end walls 300 and 302 of the lower clamp portion may include notches further receiving retention bushings 312a and 312b formed in the IV tube 214 to prevent longitudinal movement of the IV tube 214 with respect to the IV tube cartridge 220 along the longitudinal axis 222. The IV tube 214 may also pass through a spring bias clamp element 313 that automatically clamps the IV tube 214 when the IV tube cartridge 220 is separated from the upper electronics portion 218.

The rear edge 315 of the bottom wall 310 of the upper electronics portion 218 may support a hinge pin 314 spaced below the bottom wall 310 and extending generally parallel to the longitudinal axis 222 to receive downwardly extending open hinge collars 316 of the IV tube cartridge 220 spaced along the rear sidewall 306 and extending away from the longitudinally extending sidewalls 304 and 306 toward an exterior of the tray. The open hinge collars 316 may attach to and hinge about the hinge pin 314.

The front edge 318 of the bottom wall 310 of the upper electronics portion 218 may support downwardly extending hooks 320 activated by release button or lever 232. When the IV tube cartridge 220 is attached by the interconnection of open hinge collars 316 and hinge pin 314, and the bottom wall 310 of the upper electronics portion 218 is pivoted downward toward the IV tube cartridge 220, the hooks 320 may pass over and attach to corresponding longitudinally-extending tabs 322 of the front sidewall 304 of the IV tube cartridge 220 to retain the upper electronics portion 218 and IV tube cartridge 220 together with the IV tube 214 in proper alignment therebetween.

The IV tube 214 may include an IV tube connector 217 that may communicate with a typical IV line 213 that communicates with the patient 219 through a needle or port or the like, or alternatively, may communicate with a syringe 329 to fill the syringe 329 with liquid medicament for future delivery to the patient 219. The IV tube 214 may be flexible and pliable, for example, a soft silicone material or a PVC peristaltic material, to facilitate peristaltic pumping or may be a combination of a more rigid vinyl and softer central materials with the flexible central materials positioned only in the area of peristaltic plunger elements 244, and therefore may be a different material from the traditional IV line 213.

Various drug delivery protocols may be supported by the control electronics and the present invention contemplates that the ambulatory pump 210 may be comfortably worn by the patient 219 for several hours or days depending on the drug delivery protocol. In some embodiments, a single drug bolus may be delivered or drugs may be delivered periodically over an extended time.

Following drug delivery, the release button or lever 232 which may be pressed or rotated to allow release of the hooks 320 from the tabs 322 to release the upper electronics portion 218 which may be reused with a new disposable housing 212 and prefilled drug container 215.

Therefore, the present invention provides a preloaded disposable housing 212 holding a pre-filled drug container 215 from the pharmacist which further allows the patient 219 to connect the IV tube connector 217 to the prefilled drug container 215 and the upper electronics portion 218 of the ambulatory pump 210 to permit drug delivery in the home of the patient 219.

It is understood that the ambulatory pump assembly of the present invention is not limited to use for intravenous infusion therapy but may also be used for subcutaneous, arterial and epidural infusions, as for a wide range of medical purposes such as the administration of chemotherapy, pain management, total parenteral nutrition, anti-nausea, antibiotics, and the like.

Certain components of the wearable pump assembly 10 suitable for use with the present invention is described generally in US patent application 17/520,182, entitled "Clip-On Flow Control for IV Lines," filed Nov. 5, 2021; U.S. patent 10,869,963, entitled "Low-cost Ambulatory Medical Pump," filed Aug. 28, 2014; and U.S. patent 11,712,512, entitled "Ambulatory medical pump cartridge locking system," filed May 20, 2020, each of which is hereby incorporated by reference.

Certain terminology is used herein for purposes of reference only, and thus is not intended to be limiting. For example, terms such as "upper", "lower", "above", and "below" refer to directions in the drawings to which reference is made. Terms such as "front", "back", "rear", "bottom" and "side", describe the orientation of portions of the component within a consistent but arbitrary frame of reference which is made clear by reference to the text and the associated drawings describing the component under discussion. Such terminology may include the words specifically mentioned above, derivatives thereof, and words of similar import. Similarly, the terms "first", "second" and other such numerical terms referring to structures do not imply a sequence or order unless clearly indicated by the context.

When introducing elements or features of the present disclosure and the exemplary embodiments, the articles "a", "an", "the" and "said" are intended to mean that there are one or more of such elements or features. The terms "comprising", "including" and "having" are intended to be inclusive and mean that there may be additional elements or features other than those specifically noted. It is further to be understood that the method steps, processes, and operations described herein are not to be construed as necessarily requiring their performance in the particular order discussed or illustrated, unless specifically identified as an order of performance. It is also to be understood that additional or alternative steps may be employed.

References to "a microprocessor" and "a processor" or "the microprocessor" and "the processor," can be understood to include one or more microprocessors that can communicate in a stand-alone and/or a distributed environment(s), and can thus be configured to communicate via wired or wireless communications with other processors, where such one or more processor can be configured to operate on one or more processor-controlled devices that can be similar or different devices. Furthermore, references to memory, unless otherwise specified, can include one or more processor-readable and accessible memory elements and/or components that can be internal to the processor-controlled device, external to the processor-controlled device, and can be accessed via a wired or wireless network.

It is specifically intended that the present invention not be limited to the embodiments and illustrations contained herein and the claims should be understood to include modified forms of those embodiments including portions of the embodiments and combinations of elements of different embodiments as come within the scope of the following claims. All of the publications described herein, including patents and non-patent publications, are hereby incorporated herein by reference in their entireties.

To aid the Patent Office and any readers of any patent issued on this application in interpreting the claims appended hereto, applicants wish to note that they do not intend any of the appended claims or claim elements to invoke 35 U.S.C. 112(f) unless the words "means for" or "step for" are explicitly used in the particular claim.

## Claims

1. A wearable pump infusion assembly, comprising:
a housing configured to selectively support a vial containing a medicament for delivery to a patient;
a patient support member to removably mount the housing on the patient;
a bottle connector mounted within the housing, wherein the bottle connector includes:
a hollow needle to puncture a seal on the vial as the vial is inserted into the housing and to receive the medicament from the vial, and
a connector in fluid communication with the hollow needle to receive the medicament from the vial;
a flexible IV tube having a first end connected to the connector, a second end connected to another connector, and a central portion extending between the first end and the second end;
a peristaltic pump including a plurality of plunger elements selectively engaging the central portion of the flexible IV tube, wherein the plunger elements selectively compress and release the central portion of the flexible IV tube to draw the medicament from the vial.

2. The wearable pump infusion assembly of claim 1, wherein the bottle connector further comprises:
an opening defining, at least in part, a fluid communication path between an ambient environment outside the vial and an interior volume of the vial; and
a hydrophobic filter in the fluid communication path to permit air from the ambient environment into the vial and to prevent the medicament from exiting the opening.

3. The wearable pump infusion assembly of claim 1 or 2, further comprising a locking assembly to engage a neck of the vial, to draw the vial onto the hollow needle of the bottle connector, and to secure the vial within the bottle connector.

4. The wearable pump infusion assembly according to any one of the preceding claims, wherein the housing further comprises:
a first portion, comprising:
a first interlocking element,
at least one vial retaining member to receive the vial,
the bottle connector mounted within the housing, and
the flexible IV tubing mounted therein;
a second portion, comprising:
a second interlocking element, the second interlocking element complementary to the first interlocking element and wherein the first and second interlocking elements hold the first and second portions of the housing together, and
an electronics housing, wherein a controller for the wearable pump infusion assembly is contained within the electronics housing.

5. The wearable pump infusion assembly of claim 4, wherein the second portion of the housing is reusable with a plurality of first portions of the housing.

6. The wearable pump infusion assembly of claim 4, wherein the electronics housing further comprises:
at least one selection device to adjust a desired flow rate of the medicament from the vial; and
at least one flow rate sensor to detect an actual flow rate of the medicament from the vial, wherein the controller is operative to receive a feedback signal from the at least one flow rate sensor and to adjust a rate at which the plurality of plunger elements engage the central portion of the flexible IV tube as a function of the feedback signal to achieve the desired flow rate.

7. The wearable pump infusion assembly of claim 6, wherein the at least one selection device comprises at least one selection button activated by the patient to adjust the desired flow rate.

8. The wearable pump infusion assembly of claim 6, wherein the at least one selection device further comprises at least one selection switch within the electronics housing to adjust the desired flow rate.

9. A method for delivering a medicament to a patient, comprising the steps of:
inserting a vial containing the medicament for delivery to the patient into a housing, wherein a bottle connector is mounted within the housing and wherein the bottle connector comprises:
a hollow needle to puncture a seal on the vial as the vial is inserted into the housing and to receive the medicament from the vial, and
a connector in fluid communication with the hollow needle to receive the medicament from the vial;
placing a patient support member on the patient to support the housing on the patient;
pressing a button on a user interface to initiate treatment of the patient; and
delivering the medicament to the patient via a peristaltic pump and a flexible IV tube, wherein:
the flexible IV tube has a first end connected to the connector, a second end connected to another connector, and a central portion extending between the first end and the second end,
the peristaltic pump includes a plurality of plunger elements selectively engaging the central portion of the flexible IV tube to compress and release the central portion of the flexible IV tube to draw the medicament from the vial.

10. The method of claim 9, further comprising the step of drawing air from an ambient environment outside the vial into an interior volume of the vial while delivering the medicament to the patient, wherein the bottle connector further comprises:
an opening defining, at least in part, a fluid communication path between the ambient environment and the interior volume of the vial; and
a hydrophobic filter in the fluid communication path to permit the air from the ambient environment into the vial and to prevent the medicament from exiting the opening.

11. The method of claims 9 or 10 further comprising the steps of:
drawing the vial onto the hollow needle of the bottle connector with a locking assembly; and
securing the vial to the bottle connector with the locking assembly.

12. The method according to any one of the preceding claims further comprising the step of connecting a first portion of the housing to a second portion of the housing, wherein:
the first portion comprises:
a first interlocking element,
at least one vial retaining member to receive the vial,
the bottle connector mounted within the housing, and
the flexible IV tubing mounted therein;
the second portion comprises:
a second interlocking element, the second interlocking element complementary to the first interlocking element and wherein the first and second interlocking elements connect the first and second portions of the housing together, and
an electronics housing, wherein a controller for the wearable pump infusion assembly is contained within the electronics housing.

13. The method of claim 12, wherein the second portion of the housing is reusable with a plurality of first portions of the housing.

14. The method of claim 13 further comprising the steps of:
adjusting a desired flow rate of the medicament from the vial with at least one selection device present in the electronics housing;
measuring an actual flow rate of the medicament from the vial with at least one flow rate sensor; and
adjusting a rate at which the plurality of plunger elements engage the central portion of the flexible IV tube to achieve the desired flow rate, wherein the controller is operative to receive a feedback signal from the at least one flow rate sensor and to adjust the rate as a function of the feedback signal.

15. The method of claim 14, wherein the at least one selection device is either at least one selection button activated by the patient present on the electronics housing or at least one selection switch is present within the electronics housing.
